# EUROPEAN PATENT APPLICATION

(11) **EP 4 574 086 A1**
(43) Date of publication of application: **25.06.2025**
(21) Application number: 23855106.3
(22) Date of filing: 10.08.2023
(51) Int. Cl.: A61C 7/00, A61C 7/08, A61C 9/00, G06T 1/00, G06T 17/00, G16H 30/00, G16H 50/50, A61C 13/00, G06T 19/20

(54) **PROGRAM FOR DESIGNING CLEAR ORTHODONTIC APPLIANCE BY USING COMPUTER**

(30) Priority: 19.08.2022 KR 20220103848
(71) Applicant: ODS. Co. Ltd, Incheon 21990 (KR)
(72) Inventor: PARK, Sungwon, Incheon 22003 (KR); SIM, Miyoung, Incheon 22003 (KR)
(74) Representative: Valet Patent Services Limited
(86) International application number: PCT/KR2023/011893
(87) International publication number: WO 2024/039139

(57) **Abstract**

The present invention is characterized by the following steps:

Generating and storing multiple dental images sequentially configured according to each correction stage (S01), Displaying any one of the multiple dental images from the previous step (S02), After displaying the dental image in step S02, setting and displaying a reference line in the transverse direction for each tooth to determine the overall shape of the transparent orthodontic aligner (S03), Setting a cutting line corresponding to the end portion of the orthodontic aligner, which is spaced a certain distance from the gum direction based on the reference line, and displaying it on the dental image from step S02 (S04), Displaying the first treatment image, which shows the transparent orthodontic aligner partially covering both the upper and lower dental arches, based on the cutting line set in step S04(S05).

## Description

### Technical Fields

The present invention relates to a transparent orthodontic aligner used for teeth alignment, and specifically to a method and program for designing such an appliance using a computer. More specifically, the invention involves scanning the patient's oral structure using a 3D scanner to generate and store information related to the treatment images, and displaying the stored tooth images alongside the transparent orthodontic aligner to be designed, in order to design an orthodontic aligner optimized for the target teeth. The process of creating the transparent orthodontic aligner, which is directly fabricated using a 3D printer, can be divided into two main stages: the "Set-up" stage, where the target teeth are moved (or rotated) to the desired position, and the design stage, where the transparent orthodontic aligner is designed to align with these movement settings. This invention pertains to a program that designs the transparent orthodontic aligner for each movement setting stage of the target teeth.

### Background Technology

The conventional method for creating a dental orthodontic aligner is as follows:
First, the practitioner examines the patient's oral structure and creates a dental model that matches the shape of the teeth. The patient's dental model is typically made by creating a mold of the patient's oral structure, and then using plaster or similar materials to form the model. Once the plaster dental model is created, a sheet-form polyol material is heated and pressed in a press machine to form the transparent orthodontic aligner, custom-made for the patient. This method, which has been the standard, requires manual collaboration between the dentist and the dental technician, and is typically carried out by skilled dental technicians. It is time-consuming and labor-intensive, resulting in increased manufacturing costs for the dental appliance. Recently, however, methods utilizing 3D scanners to scan the patient's oral structure and store the information for creating a dental model have become widely used. This method is a more advanced approach compared to the traditional method of taking direct impressions of the teeth to create a dental model. In other words, in dental practices, the 3D scanning data of the patient's oral structure is sent to a dental laboratory, where a dental model is made based on the received data, and the transparent orthodontic aligner is then pressed according to the model. The methods described above all involve first creating a dental model and then producing the orthodontic aligner based on it. In contrast, the present invention does not require a dental model, as it uses a 3D printer to directly print the orthodontic aligner. This invention relates to a program for designing a transparent orthodontic aligner that is directly manufactured using a 3D printer.

### Detailed description of the Invention

### Technical Challenges

The program for designing a transparent orthodontic aligner using a computer, according to the present invention, aims to dramatically reduce the time and effort required to manufacture the orthodontic aligner by directly printing it with a 3D printer, without the need to create a separate dental model. This results in a significant reduction in the cost of the orthodontic aligner. Additionally, the program aims to simplify the manufacturing process of the orthodontic aligner by allowing the direct production of the appliance in the dental clinic, without the need for the laborious collaboration between the dental clinic and the dental laboratory.

Furthermore, the program for designing a transparent orthodontic aligner according to the present invention provides the optimal shape, thickness, and inner surface gap of the transparent orthodontic aligner for each setup stage, in order to ensure the optimal corrective force. This increases the comfort and effectiveness of the transparent orthodontic aligner produced using a 3D printer. The program for designing a transparent orthodontic aligner using a computer according to the present invention proceeds with the design of the transparent orthodontic aligner while overlapping the image of the appliance with the patient's tooth arrangement (hereinafter referred to as 'dentition') at each movement setting (setup) stage. This approach enables more precise and easier production of the transparent orthodontic aligner, which is the technical goal of the invention.

### Technical Solution

The present invention is devised to solve the technical problems described above and includes the following steps:
1. Generating and storing multiple dentition images where the target teeth are sequentially moved according to each correction stage (S01).
2. Displaying any one of the multiple dentition images from the previous step (S02).
3. In the dentition image displayed in step S02, setting a reference line for each tooth in the lateral direction to determine the overall shape of the transparent orthodontic aligner, and displaying it (S03).
4. Setting a cut line corresponding to the end part of the orthodontic aligner, which is formed by being spaced a certain distance from the reference line toward the gum direction, and displaying it in the dentition image displayed in step S02 (S04).
5. Displaying the first treatment image, which shows the transparent orthodontic aligner, semi-transparently covering the upper and lower dentition according to the cut line set in step S04 (S05).
6. Inputting the overall gap value between the dentition and the transparent orthodontic aligner in the first treatment image (S06).
7. Displaying a new second treatment image reflecting the gap value entered in step S06 (S07).
8. Selecting the teeth to be corrected in the dentition, and modifying the gap value between the teeth and the inner surface of the transparent orthodontic aligner to a smaller value than the gap value entered in step S06, for the selected teeth in the orthodontic aligner (S08).
9. Displaying a new third treatment image reflecting the modified gap value from step S08 (S09).
10. Inputting the overall thickness value of the transparent orthodontic aligner (S10).
11. Displaying a new fourth treatment image reflecting the thickness value entered in step S10 (S11).
12. Selecting the teeth to be corrected in the dentition again, and modifying the thickness value for the selected teeth in the orthodontic aligner to a value greater than the thickness value entered in step S10 (S12).
13. Displaying a new fifth treatment image reflecting the modified thickness value from step S12 (S13).
14. Storing the fifth treatment image generated in step S13 (S14).

### Effects of the Invention

The program for designing a transparent orthodontic aligner using a computer, according to the present invention, significantly reduces the time and effort required for manufacturing the orthodontic aligner by directly printing it with a 3D printer, without the need to create a separate dental model. This greatly improves the production efficiency of the orthodontic aligner, enhancing the economic feasibility of the product. Furthermore, the program drastically reduces the collaboration between the dental clinic and the dental laboratory, allowing for in-house manufacturing of the orthodontic aligner directly at the dental clinic. This simplifies the manufacturing process of the appliance. The program for designing a transparent orthodontic aligner according to the present invention enables the easy design of the optimal orthodontic aligner by changing the shape of the transparent appliance while viewing a composite treatment image where the dentition and the transparent orthodontic aligner are overlapped at each movement setting (Setup) stage. The updated treatment image can be visually confirmed in real-time, making it possible to design the appliance that perfectly fits the target teeth. Moreover, by presenting the optimal shape, thickness, and inner surface gap between the teeth and the transparent orthodontic aligner for each setup stage, the program increases the comfort and effectiveness of the transparent orthodontic aligner produced using a 3D printer, thereby improving both its usability and utility.

### Brief Description of the Drawings

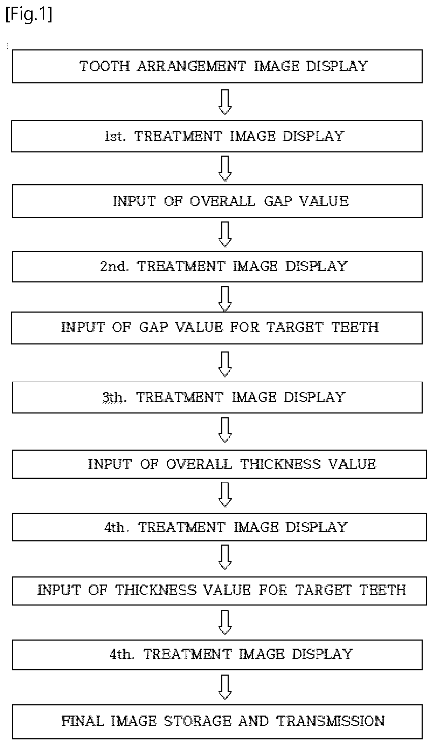

Fig. 1 illustrates the design sequence of the transparent orthodontic aligner according to the present invention.
Fig. 2 illustrates the design sequence of the attachment according to the present invention.
Fig. 3 shows the change in corrective force over time based on the thickness of the transparent orthodontic aligner.
Fig. 4 shows the transparent orthodontic aligner with a button-type attachment formed according to the present invention.
Fig. 5 shows the transparent orthodontic aligner with a hook-type attachment formed according to the present invention.
Fig. 6 illustrates an embodiment of the transparent orthodontic aligner according to the present invention.
Fig. 7 illustrates another embodiment of the transparent orthodontic aligner according to the present invention.

### Best Mode for Carrying Out the Invention

The present invention includes the following steps to solve the technical problems:
Generating and storing multiple dentition images where the target teeth are sequentially moved according to each correction stage (S01).
Displaying any one of the multiple dentition images from the previous step (S02).
In the dentition image displayed in step S02, setting a reference line for each tooth in the lateral direction to determine the overall shape of the transparent orthodontic aligner, and displaying it (S03).
Setting a cut line corresponding to the end part of the orthodontic aligner, which is formed by being spaced a certain distance from the reference line toward the gum direction, and displaying it in the dentition image displayed in step S02 (S04).
Displaying the first treatment image, which shows the transparent orthodontic aligner, semi-transparently covering the upper and lower dentition according to the cut line set in step S04 (S05).
Inputting the overall gap value between the dentition and the transparent orthodontic aligner in the first treatment image (S06).
Displaying a new second treatment image reflecting the gap value entered in step S06 (S07).
Selecting the teeth to be corrected in the dentition, and modifying the gap value between the teeth and the inner surface of the transparent orthodontic aligner to a smaller value than the gap value entered in step S06, for the selected teeth in the orthodontic aligner (S08).
Displaying a new third treatment image reflecting the modified gap value from step S08 (S09).
Inputting the overall thickness value of the transparent orthodontic aligner (S10).
Displaying a new fourth treatment image reflecting the thickness value entered in step S10 (S11).
Selecting the teeth to be corrected in the dentition again, and modifying the thickness value for the selected teeth in the orthodontic aligner to a value greater than the thickness value entered in step S10 (S12).
Displaying a new fifth treatment image reflecting the modified thickness value from step S12 (S13).

### Form for implementing the invention

Hereinafter, the preferred embodiments of the present invention will be described in detail with reference to the accompanying drawings. The advantages, features, and methods of achieving them will become clear by referring to the embodiments described below, along with the accompanying drawings. However, the present invention is not limited to the embodiments presented here and can be implemented in various other forms. These embodiments are provided merely to ensure that the description of the invention is complete and to fully inform those skilled in the art of the scope of the invention. The present invention is defined only by the scope of the claims. The present invention relates to a program for designing a transparent orthodontic aligner using a computer. In this context, "computer" includes various devices that perform computational processing and provide results to the user, and it is composed of input processing units, processing units, output units, and so on.

In this specification, the term "treatment image" refers to a two-dimensional, three-dimensional, or multi-dimensional image showing the overall arrangement of the teeth. It includes images obtained through medical imaging techniques such as tomography. For example, CT (Computer Tomography) images, MRI (Magnetic Resonance Imaging) images, PET (Positron Emission Tomography) scans, CBCT (Cone Beam CT), and oral scanner images are included. Moreover, the "treatment image" includes not only the original captured images but also 2D or 3D images modified by additional reconstruction techniques. In this specification, the term "target teeth" refers to teeth that are improperly positioned or abnormally rotated, requiring movement and/or rotation through orthodontic treatment.

In this specification, the term "lingual surface" refers to the side of the tooth that contacts the tongue, the term "labial surface" refers to the side of the tooth that contacts the lips, the term "buccal surface" refers to the side of the tooth that contacts the inner surface of the cheek, the term "occlusal surface" refers to the top surface of molars and other teeth that directly engage in chewing food, and the term "incisal surface" refers to the slanted inner surface of teeth that do not have an occlusal surface (e.g., the incisors), which serves the function of cutting food. Meanwhile, the term "buccal bulge" refers to the area on the lateral surface of the tooth where the transverse width of the tooth is at its maximum.

Hereinafter, the embodiments of the present invention will be described in detail with reference to the accompanying drawings.

Step S01 involves generating and storing dentition images that are sequentially moved according to each orthodontic stage. Orthodontic treatment involves gradual movement/rotation/intrusion/extrusion of teeth over a long period of time. During the treatment period, as the teeth gradually shift, the transparent orthodontic aligner must be continuously and sequentially replaced and worn. In other words, the orthodontic treatment progresses through several stages, and a corresponding orthodontic aligner is required for each stage. For example, if the orthodontic treatment is set to 20 stages, the correction goal for each stage will be set by the practitioner at each of the 20 stages over time. As a result, 20 dentition images corresponding to the 20 stages will be generated.

The 3D scanner scans the patient's dentition images and stores them. These stored dentition images are then sent to a computer and displayed. In the displayed dentition image, the practitioner generates a dentition image corresponding to the correction goal for each stage. This process is referred to as the "setup" in the present invention. In other words, the "setup" refers to establishing the corrected dentition state for each stage. Thus, Step S01 involves generating and storing several dentition images based on the practitioner's setup, and Step S02 involves selecting and displaying any one of these stored dentition images.

Step S03 involves setting a reference line in the lateral direction for each tooth to determine the overall shape of the transparent orthodontic aligner, and then displaying it. The reference line can be created, connected, extended, interrupted, or adjusted according to the tooth. The reference line can be considered as the setting line that defines the extent to which the transparent orthodontic aligner will cover the tooth. For the target teeth, depending on the area where the correction force is applied, the reference line could be at the tooth's edge, the connecting line at the bulging part (the point where the tooth's circumference is the largest), or the boundary line with the gums.

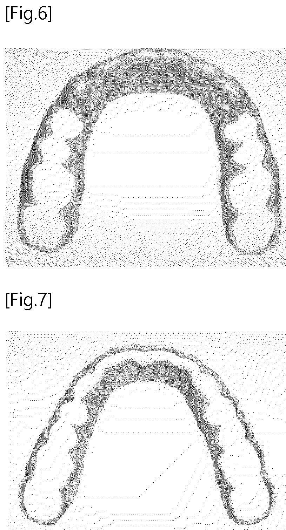

FIG. 6 shows one embodiment of the transparent orthodontic aligner according to the present invention, and FIG. 7 shows another embodiment of the transparent orthodontic aligner according to the present invention.

For example, in the case of teeth that do not require correction, the reference line may be set at the tooth's edge or may be partially generated, so that the transparent orthodontic aligner can be omitted for those teeth. FIG. 6 illustrates the case where no correction is required for the molars on both the left and right sides of the dentition, and the orthodontic aligner for those parts is omitted. FIG. 7 shows the case where no orthodontic aligner is required on the upper part of the entire dentition, and the appliance for that part is omitted. On the other hand, when the bulging part of the target tooth is set as the reference line, the spacing value, thickness value, etc., can be set differently for the upper and lower parts based on the reference line to satisfy the required correction for each tooth. Additionally, when a reference line dividing the left and right sides of the target tooth is created, the spacing value, thickness value, etc., can be set differently for the left and right parts based on the reference line to satisfy the required correction for each tooth.

Step S04 involves forming the incision line. The incision line is set on the monitor and is used to define the edge of the transparent orthodontic aligner. The incision line is formed at a specific distance from the reference line towards the gum direction, and it can be set at various values, such as 1mm, 2mm, 5mm, etc. The position of this incision line will be determined by considering factors such as the required correction force for the specific tooth, the ease of wearing the transparent orthodontic aligner, and other relevant considerations.

Step S05 involves displaying the first treatment image, where the transparent orthodontic aligner is displayed as covering the entire dentition in the displayed dentition image. The transparent orthodontic aligner, which is shown as semitransparent, is displayed covering both the upper and lower dentition according to the incision line set in Step S04. The display can show both the upper and lower dentition simultaneously, or each can be displayed separately. The first treatment image displays the state where the transparent orthodontic aligner is placed on the teeth (i.e., the image of the transparent orthodontic aligner is overlapped on the tooth image). This corresponds to the initial stage of designing the transparent orthodontic aligner.

Step S06 involves entering the gap value between the teeth and the transparent orthodontic aligner in the first treatment image, and Step S07 involves displaying the new second treatment image reflecting the entered gap value. The gap between the teeth and the transparent orthodontic aligner directly affects the orthodontic force. If the gap is too small (i.e., when there is almost no space between the teeth and the appliance), the appliance may not fit well, or it could cause severe pain for the patient during the correction process. On the other hand, if the gap becomes too large (i.e., when the space between the teeth and the appliance increases), the appliance may be easier to wear, but the correction force will be insufficient. Step S06 is the phase where the overall gap between the teeth and the transparent orthodontic aligner is set, considering the comfort of wearing the appliance and the orthodontic force. This step corresponds to the initial design process of the transparent orthodontic aligner.

Step S08 involves adjusting and entering a smaller gap value between the teeth and the inner surface of the transparent orthodontic aligner to apply the optimal orthodontic force and direction of force for the target teeth. Step S09 follows by displaying a new third treatment image reflecting the gap value entered in Step S08. For the part of the transparent orthodontic aligner that contacts the target tooth, the gap needs to be narrower compared to other areas. This adjustment ensures that the appliance fits more closely to the tooth, allowing it to apply the desired orthodontic force effectively. The gap value can be entered in various ways, such as through a menu displayed on the screen where the user clicks a button and enters numbers, or through a pop-up window that appears on the screen to input the values. Alternatively, the user may adjust the gap by dragging the transparent orthodontic aligner with the mouse on the screen. Once the gap value between the target tooth and the transparent orthodontic aligner is entered, the third treatment image is immediately displayed on the screen according to the input values. The practitioner can visually confirm whether the gap between each tooth and the appliance is properly set. If any errors are found, the gap value can be promptly re-entered, allowing for real-time corrections. This instant feedback mechanism ensures that adjustments can be made quickly and accurately during the design process.

To correct a tooth through movement, rotation, intrusion, or extrusion, specific forces must be applied depending on the desired result. For example, when rotation correction is required for a target tooth, a moment must be applied to rotate the tooth to the target angle. To apply a rotational moment to the target tooth, forces are needed in the direction of pushing adjacent teeth horizontally on the lingual (inner) and labial (outer) surfaces (or buccal surface for molars). In order for this correction force to be effective, the gap value may need to be reduced in the direction of pushing the tooth (S201), or the thickness value may need to be increased (S202) in those areas. Essentially, to move the tooth in a particular direction, smaller gap values are input for those specific parts compared to the overall gap settings. Therefore, S201 and S202 represent the process where, for teeth requiring rotational correction, the gap or thickness values are adjusted to be smaller or larger than initially set in the direction needed to push the tooth, ensuring that the necessary correction forces are applied.

Steps S301 and S302 refer to the design stages of the transparent orthodontic aligner for teeth requiring extrusion correction.

In cases where extrusion correction is required for the target tooth, an extrusion force must be applied to the tooth. To apply this extrusion force, it is necessary to set the gap value smaller in the direction of the gum from the reference line corresponding to the tooth's widest point (the buccal prominence). In this case, starting from the reference line at the buccal prominence, the gap value in the gum direction should be set 0.1 to 0.5 mm smaller than the gap value at the tooth's tip. If the difference in the gap value is larger than this range, the desired extrusion force will not be achieved. On the other hand, if the difference is smaller than this range, it will cause a significant issue with the wearability of the appliance, making it very difficult to wear. This extrusion correction method is unique to this invention and cannot be expected from conventional sheet compression methods used in transparent orthodontic aligners (where the compression process may result in undercuts at the tooth-gum junction). This method provides a unique functional effect specific to the present invention.

Step S10 involves entering the overall thickness value of the transparent orthodontic aligner, while Step S11 involves displaying a new fourth treatment image, reflecting the thickness value entered in Step S10. This step is part of the process of determining the overall thickness of the transparent orthodontic aligner. By displaying the appliance, the practitioner can visually assess the appropriateness of the thickness in real time, making adjustments as necessary to ensure the overall thickness of the appliance is suitable. This process allows for a more accurate and effective design of the appliance.

Step S12 involves separately modifying and inputting the thickness value for the area of the transparent orthodontic aligner that comes into contact with the orthodontic target teeth. Step S13 then involves displaying a new fifth treatment image reflecting the thickness value entered in Step S12.

In order to apply pressure to the target teeth and induce movement (or rotation) of the teeth, the thickness of the transparent orthodontic aligner must exceed a certain value. This thickness is necessary for the appliance to generate the required corrective force. However, if the appliance's thickness is too large, it can significantly reduce wearability, causing discomfort for the patient. Additionally, increasing the thickness beyond a certain point does not provide any further corrective effect, meaning that while the thickness needs to be sufficiently large to create corrective force, it must not be excessive to avoid compromising comfort and effectiveness.

The correction method using transparent orthodontic aligners works by gradually shifting the position or rotation of the teeth towards the target. Once the teeth reach the desired position or rotation, the transparent orthodontic aligner is replaced with the next stage of the appliance. This newly replaced appliance then continues the correction process for the next stage. Therefore, at regular intervals, the transparent orthodontic aligners need to be replaced in stages to facilitate continuous orthodontic treatment.

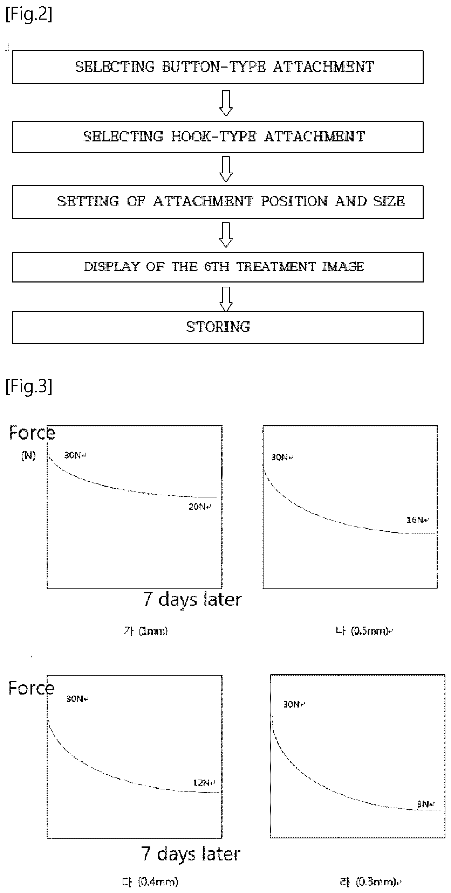

Figure 3 shows the change in orthodontic force over time according to the thickness of the transparent orthodontic aligner. The minimum thickness (the part that contacts the teeth) required to maintain a certain level of orthodontic force during the typical replacement cycle of 7 to 14 days is 0.3mm. If the thickness exceeds 1mm, patients tend to experience significant discomfort, and no further orthodontic force is effectively generated. Therefore, it is desirable for the thickness of the transparent orthodontic aligner in contact with the target teeth to be greater than 0.3mm but not exceed 1mm. More preferably, a thickness in the range of 0.3mm to 0.7mm is recommended.

Additionally, as previously explained, in cases requiring extrusion correction, adjustments to the gap values can be made to introduce extrusion force. The thickness can also be increased by a specific amount in the direction of the gums, based on the reference line from the tooth's prominence, to introduce extrusion force to the target tooth. For example, the thickness value could be increased by 1 to 2mm in the gum direction from the prominence reference line. This creates a difference in the orthodontic force across the upper and lower parts of the tooth, inducing extrusion force, and the transparent orthodontic aligner of the present invention thus provides extrusion correction effects. The input method for the thickness value is the same as the gap value input method (via menu settings or a pop-up window), which has already been explained, so further details on the input method will be omitted here.

S13 step is the stage where the 5th treatment image generated in step S12 is saved, and in step S14, the saved 5th treatment image and the 6th treatment image saved in step S105 are transmitted to the patient's device. The patient can preview the transparent orthodontic aligner they will wear in 2D or 3D images through their device (it may be required to install a specific app beforehand).

The 5th treatment image saved in step S14 may be a 2D image, such as a cross-sectional view, or it could be a 3D image. These images are then sent to a 3D printer or similar device and used to manufacture the transparent orthodontic aligner.

On the other hand, in cases where the patient has a severe malocclusion or similar issues, there may be a need to secure additional orthodontic force beyond the adjustments of the spacing and thickness of the transparent orthodontic aligner.

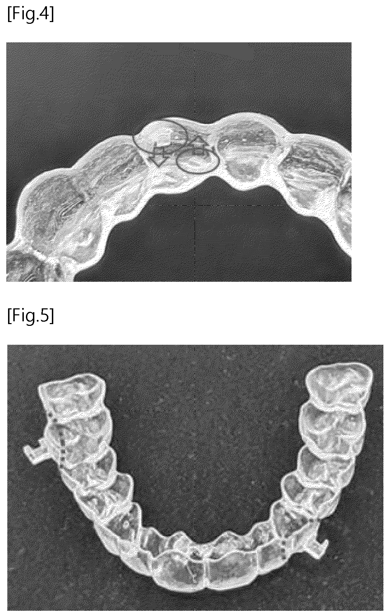

Step S101 is the stage where the practitioner decides whether to add button-type attachments on the inner surface of the transparent orthodontic aligner that contacts the teeth. As shown in Figure 4, the button-type attachment is a protruding shape that forms on the inner surface of the transparent orthodontic aligner. This attachment helps generate stronger orthodontic forces on the target teeth, enabling more effective correction. When additional orthodontic force is required beyond the conventional force, selecting the button-type attachment allows for a transparent orthodontic aligner design with enhanced correction power. Figure 4 illustrates a transparent orthodontic aligner with an attachment installed on the inner surface to apply a rotational moment to the target tooth, thus inducing tooth rotation correction. The attachments are installed on opposite sides of the transparent orthodontic aligner, applying a rotational moment to the tooth. The positioning of these attachments can be varied depending on the target tooth's environment, and Figure 4 is just one example of how the design can be adapted to the needs of the specific case.

Step S102 is the stage where the practitioner decides whether to add hook-type attachments on the outer surface of the transparent orthodontic aligner that contacts the teeth. In cases where additional orthodontic force is required beyond the conventional force for the target teeth, as shown in Figure 5, hook-type attachments may be used on the outer surface of the transparent orthodontic aligner. Unlike the button-type attachments mentioned earlier, hook-type attachments are formed on the outer surface of the transparent orthodontic aligner. Of course, in some cases, both types of attachments can be used simultaneously, depending on the specific needs of the patient and the treatment requirements.

Step S103 is the stage where the shape, size, and attachment position of the button-type and hook-type attachments selected in steps S101 and S102 are set. Step S104 is the stage where the final, modified sixth treatment image is displayed.

For example, the shape of the button-type attachment can be modified into various forms such as round, square, or diamond, and its size (cross-sectional area) can also be modified depending on the environment of the target tooth. Such modifications fall within the technical scope of the present invention. In Step S103, if the use of button-type and/or hook-type attachments is decided, the process involves determining their size (e.g., protrusion degree, surface area) and position. Once this process is complete, the sixth treatment image is finalized and saved. As explained earlier, the button-type and hook-type attachments can be modified in various ways in terms of their types and shapes, which is evident to those skilled in the art. It is also possible to create a separate folder listing various shapes and sizes of button-type and hook-type attachments and, when necessary, open the folder and overlay the design onto the display screen. Therefore, these modifications are considered simple variations of the present invention and do not deviate from the technical essence of the invention, and naturally, they fall within the scope of the invention's claims.

The program according to the present invention is a computing program (also referred to as a program, software, software application, or code) that includes machine instructions for a programmable processor and can be implemented using high-level process and/or object-oriented programming languages and/or assembly/machine languages. Each step described in this application may be performed in parallel, sequentially, or in a different order, and the disclosed technology does not specifically limit the order as long as the intended result can be achieved. The specific embodiments described do not limit the scope of protection of this application. A person skilled in the art can make various modifications, combinations, sub-combinations, and alternatives depending on design requirements and other factors. Any modifications, equivalent substitutions, or improvements made within the spirit and principles of this application are all considered to fall within the scope of protection of this application.

## Claims

1. A program for designing a transparent orthodontic aligner using a computer, **characterized by** executing the following steps:
A step (S01) of generating and storing multiple dental images in which the target teeth sequentially move according to each orthodontic stage;
A step (S02) of displaying any one of the multiple dental images generated in the step S01;
A step (S03) of setting a baseline in the transverse direction of each tooth in the displayed dental image from step S02, in order to determine the overall shape of the transparent orthodontic aligner, and displaying it;
A step (S04) of setting an incision line corresponding to the end portion of the transparent orthodontic aligner, formed by being spaced by a predetermined distance from the baseline in step S03, and displaying it;
A step (S05) of displaying a first treatment image in which the transparent orthodontic aligner, which covers the upper and lower dental arches according to the incision line set in step S04, is displayed in a translucent state;
A step (S06) of inputting the overall gap value between the dental arch and the transparent orthodontic aligner in the first treatment image from step S05;
A step (S07) of displaying a new second treatment image reflecting the gap value input in step S06;
A step (S08) of selecting the target teeth from the upper and lower dental arches, and modifying the gap value to a smaller value than the gap value input in step S06 for the part of the transparent orthodontic aligner that contacts the selected teeth;
A step (S09) of displaying a new third treatment image reflecting the modified gap value from step S08;
A step (S10) of inputting the overall thickness value of the transparent orthodontic aligner;
A step (S11) of displaying a new fourth treatment image reflecting the thickness value input in step S10;
A step (S12) of selecting the target teeth from the upper and lower dental arches, and modifying the thickness value to a larger value than the thickness value from step S10 for the part of the transparent orthodontic aligner that contacts the selected teeth;
A step (S13) of displaying a new fifth treatment image reflecting the modified thickness value from step S12;
A step (S14) of storing the fifth treatment image generated in step S13.

2. A program for designing a transparent orthodontic aligner using a computer, **characterized by** executing the following steps:
Executing all the steps from S01 to S14 according to claim 1;
A step (S101) of selecting whether to add a button-type attachment that is attached to the inner surface of the transparent orthodontic aligner in contact with the target teeth;
A step (S102) of selecting whether to add a hook-type attachment that is attached to the outer surface of the transparent orthodontic aligner in contact with the target teeth;
A step (S103) of setting the shape, size, and attachment position of the selected button-type attachment and the hook-type attachment when it has been decided to add either or both in steps S101 and S102;
A step (S104) of displaying the modified sixth treatment image according to step S103;
A step (S105) of storing the sixth treatment image generated in step S104.

3. A program for designing a transparent orthodontic aligner using a computer, according to claim 1 or 2, **characterized in that**:
The baseline is formed as follows:
In the case of the upper dental arch, the baseline is formed at one of the following points: the lower end of the tooth, the point where the tooth's bulging part is located, or the boundary line between the tooth and the gum,
In the case of the lower dental arch, the baseline is formed at one of the following points: the upper end of the tooth, the point where the tooth's bulging part is located, or the boundary line between the tooth and the gum.

4. A program for designing a transparent orthodontic aligner using a computer, according to claim 3, **characterized in that**:
It includes:
A step (S201) of reducing the set gap value in the direction that pushes the tooth for the part of the orthodontic target tooth requiring rotational correction, or
A step (S202) of increasing the set thickness value for that part.

5. A program for designing a transparent orthodontic aligner using a computer, according to claim 3, **characterized in that**:
For the part of the orthodontic aligner that contacts the tooth requiring extrusion correction:
A step (S301) of further reducing the gap value for the portion corresponding to the tooth's bulging part from the baseline in the gum direction, up to a length corresponding to 2 mm;
A step (S302) of displaying the gap value modified in step S301.

6. A program for designing a transparent orthodontic aligner using a computer, according to claim 4, **characterized in that**:
In step S12, the input thickness value is determined to be within the range of 0.3 mm to 0.7 mm.

7. A program for designing a transparent orthodontic aligner using a computer, according to claim 5, **characterized in that**:
In step S12, the input thickness value is determined to be within the range of 0.3 mm to 0.7 mm.

8. A program for designing a transparent orthodontic aligner using a computer, according to claim 6 or 7, **characterized in that**:
The fifth and sixth treatment images stored in steps S14 and S105 are further transmitted to the patient's terminal in step S15.
